# EUROPÄISCHE PATENTANMELDUNG

(11) **EP 3 207 896 A1**
(43) Veröffentlichungstag der Anmeldung: **23.08.2017**
(21) Anmeldenummer: 16156270.7
(22) Anmeldetag: 18.02.2016
(51) Int. Cl.: A61B 18/20

(54) **VORRICHTUNG ZUR BESTRAHLUNG DER HAUT**

(71) Anmelder: Fatemi, Afschin, 40474 Düsseldorf (DE)
(72) Erfinder: Dr. Fatemi, Afschin, 40474 Düsseldorf (DE)
(74) Vertreter: Roth, Andy Stefan

(57) **Zusammenfassung**

Beschrieben werden eine Vorrichtung (18) sowie ein Verfahren zur Bestrahlung einer Zielstruktur (1) mit einer Laserstrahlung (4) sowie eine geeignete Verwendung eines mit der Laserstrahlung (4) erzeugten Brennflecks oder Brennpunkts (6). Die Vorrichtung (18) verfügt über wenigstens eine Laserlichtquelle (2) und zumindest ein Optikelement (3), mittels dem die Laserstrahlung (4) durch eine Lichtaustrittsöffnung (5) geleitet und in einem Brennpunkt (6)außerhalb der Lichtaustrittsöffnung (5) fokussiert wird. Ferner ist wenigstens ein Stellelement (7) vorgesehen, mit dem der Abstand zwischen einer Hautoberfläche (8) und dem im Bereich der Zielstruktur (1) positionierten Brennpunkt (6) einstellbar ist.

Die beschriebene technische Lösung zeichnet sich dadurch aus, dass das Optikelement (3) eine Anordnung eines konvexen Spiegels (9) innerhalb eines Hohlspiegels (10) aufweist, die derart angeordnet sind, dass von der Laserlichtquelle (2) emittierte Laserstrahlung (4) zumindest teilweise durch eine Öffnung (11) im Hohlspiegel (10) hindurchtritt, die durch die Öffnung (11) hindurchgetretene Laserstrahlung (4) auf den konvexen Spiegel (9) trifft, vom konvexen Spiegel (9) zum Hohlspiegel (10) gelenkt wird und vom Hohlspiegel (10) kommend die Lichtaustrittsöffnung (5) durchquert.

## Beschreibung

Die Erfindung betrifft eine Vorrichtung sowie ein Verfahren zur Bestrahlung einer Zielstruktur mit einer Laserstrahlung sowie eine geeignete Verwendung eines mit der Laserstrahlung erzeugten Brennflecks. Die Vorrichtung verfügt hierbei über wenigstens eine Laserlichtquelle und zumindest ein Lichtlenkmittel, die derart ausgeführt sind, dass Laserstrahlung über unterschiedliche Strahlengänge in die Zielstruktur gelangt, wobei aufgrund eines durch die Bestrahlung bedingten Energieeintrags zumindest bereichsweise eine Eigenschaftsveränderung der Zielstruktur erfolgt.

Die von einem Laser ausgesandten Strahlen zeichnen sich vor allem durch ihre hohe Intensität, den sehr engen Frequenzbereich der Strahlung und damit einhergehende targetspezifischen Selektivität, eine scharfe Bündelung des Strahls sowie die große Kohärenzlänge aus. Neben vielen bekannten Anwendungen im technischen Bereich, findet der Laser auch vielfach in der Medizintechnik Anwendung. So werden beispielsweise in der Dermatologie mit Hilfe von Laserstrahlen Schnitte und Verödungen durchgeführt. Ebenso können Blutgefäße durch Laser bestimmter Wellenlänge koaguliert werden und Pigmentflecken mit Hilfe ablatierender, bzw. sogenannter schälender Laser abgetragen oder selektiv zerstört werden. Ferner werden subkutane Pigmente mit Hilfe einer ultrakurzgepulsten Laserquelle zerstört und damit entfernt, ohne die Hautoberfläche nachhaltig zu verletzen, oder mit Hilfe von langgepulsten Lasern Haarwurzeln durch Epilation dauerhaft zerstört. Darüber hinaus werden Laser teilweise zur gezielten Behandlung entzündlicher Hauterkrankungen, wie etwa der Psoriasis (Schuppenflechte), eingesetzt oder es werden oberflächliche Unebenheiten der Haut, wie Knötchen oder Fältchen, zur kosmetischen Verbesserung des Hautbildes geglättet (Resurfacing). Gemäß einer weiteren Anwendung in der Dermatologie werden Laser verwendet, um selektiv dermale Anteile zu erwärmen und so den Kollagenaufbau zu fördern und die Haut in diesem Bereich zu straffen (Subsurfacing).

Je nach der Art des verwendeten Lasers kommt es zu unterschiedlichen Wechselwirkungen zwischen der Haut bzw. dem Gewebe und dem vom Laser ausgesendeten Licht. Die Art der Wechselwirkung hängt einerseits von den optischen Eigenschaften der Haut bzw. des Gewebes, insbesondere dem Streu- und dem Absorptionskoeffizienten sowie der Dichte, und andererseits von den physikalischen Parametern des Laserlichts, insbesondere der Wellenlänge, der Energiedichte, der Wiederholrate, der Bestrahlungsdauer sowie der Spotgröße, ab.
Die Laser-Gewebe-Wechselwirkungen werden in verschiedene Mechanismen eingeteilt. Hierzu gehören die photo-thermale, photo-mechanische bzw. photo-akustische und die photo-chemische Wechselwirkung. Weiterhin wird teilweise noch das Phänomen der photo-vermittelten Ablation, die sogenannte Photoablation, genutzt. Unter diesen Effekten ist die am meisten quantifizierbare und zumeist beobachtende Reaktion der photothermische Effekt, der durch die Einbringung hoher Energien und die dadurch verursachte Verdampfung von Wasser im Gewebe erreicht wird. Je nach Beschaffenheit des bestrahlten Mediums und der entsprechenden Struktur sowie der Bestrahlungsparameter treten die verschiedenen Effekte unterschiedlich stark auf, wobei angenommen wird, dass die absorbierte Energie und die Bestrahlungszeit die Gewebewirkung zum größten Teil beeinflussen. Die entsprechenden Wechselwirkungen werden im Detail in "C. Raulin, S. Kasei (Hrsg.), Lasertherapie der Haut, Springer-Verlag Berlin, Heidelberg 2013" beschrieben.

In diesem Zusammenhang wird in der US 2011/0313408 A1 ein Laser zur Behandlung der Haut beschrieben. Wesentlich an der beschriebenen technischen Lösung ist, dass für die Bestrahlung ein erster Laserstrahl mit langer Pulsdauer und ein zweiter Laserstrahl mit kurzer Pulsdauer unter gleichzeitigem Einsatz eines Kühlelements zum Kühlen eines Oberflächenbereichs der Haut verwendet werden. Mit dem ersten Laserstrahl wird ein Volumen von Hautgewebe unterhalb des gekühlten Oberflächenbereichs erwärmt, um das Hautgewebe noch unterhalb der Hautgewebeschädigungsschwelle zu modifizieren. Der zweite Laserstrahl wird in eine Vielzahl von getrennten Laserstrahlen mit Hilfe von getrennten optischen Fasern aufgeteilt und über getrennte Wege durch das Hautgewebe in das vorerwärmte Hautgewebe gelenkt, um hier in gezielt ausgewählten, kleinen Volumina mechanische Beschädigungen hervorzurufen.

Ferner ist aus der US 2007/0239147 A1 ein System zur Behandlung von dermatologischen Erkrankungen bekannt, das auf der thermischen Schädigung einer Zielstruktur beruht. Hierbei wird gezielt ein Strahlenbündel auf einen Zielpunkt auf der Hautoberfläche gerichtet, um die Zielstruktur im Gewebe unterhalb dieser Zielposition zu schädigen. Insbesondere sind Lichtlenkmittel vorgesehen, mit denen ein zweiter Lichtstrahl auf die gleiche Zielposition gelenkt wird, um ein anderes Gewebevolumen unterhalb des Zielortes thermisch zu schädigen.

Aus der US 7,066,929 B1 ist ferner eine selektive Photothermolyse bekannt, mit der subkutanes Gewebe durch Verwendung einer Mehrzahl von Strahlen von schmalbandigen, elektromagnetischen Wellen zerstört wird. Da jeder der Strahlen nicht über die ausreichende Energie verfügt, um das Gewebe auf die benötigte Temperatur zu erwärmen und durch Übererhitzung zu zerstören, werden die einzelnen Strahlen am Zielpunkt derart überlappt, so dass ausreichende Wärme erzeugt wird, um das Zielgewebe zu zerstören. Gemäß der beschriebenen technischen Lösung ist hierfür eine Strahllenkeinrichtung vorgesehen, die die einzelnen Lichtstrahlen derart lenkt, dass jeder der Strahlen beim Verlassen der Strahlungsvorrichtung in einem anderen Winkel austritt. Mit Hilfe einer geeigneten Steuerung des Lasers werden die unterschiedlichen einzelnen Strahlen schließlich in einem Punkt fokussiert.

Im Weiteren beschreibt die WO 2013/156421 A1 ebenfalls eine Vorrichtung zur Behandlung von Haut- und/oder Gewebeschichten mit Hilfe von Laserlicht. Wesentlich an der beschrieben Vorrichtung ist, dass eine Vielzahl von Laserlichtquellen verwendet wird, um gleichzeitig oder abwechselnd eine Zielstruktur aus unterschiedlichen Richtungen zu bestrahlen. Die Laserlichtquellen und/oder geeignete Lichtlenkmittel sind derart angeordnet, dass das Laserlicht auf unterschiedlichen Wegen in die zu behandelnde Hautoder Gewebeschicht und im Bereich der Zielstruktur punktuell fokussiert wird.

Problematisch an den bekannten und derzeit zum Einsatz kommenden technischen Lösungen zur Behandlung der Haut ist oftmals, dass der Hautbereich, auf den die Laserstrahlung auftrifft, um von dort aus weiter in die Haut bzw. das subkutane Gewebe einzudringen, geschädigt wird. Derartige Schädigungen sind regelmäßig nicht erwünscht und können teilweise zu nicht unerheblichen Komplikationen führen. Weiterhin stellt es in vielen Fällen ein erhebliches Problem dar, gezielt größere, insbesondere subkutan in vergleichsweise großer Tiefe gelegene Zielstrukturen zu zerstören, ohne dass es zu Schädigungen von angrenzenden Gewebebereichen, Drüsen oder anderen Teilen der Haut oder des Gewebes kommt. Dies ist vor allem darauf zurückzuführen, dass es mit den bekannten technischen Lösungen nicht in ausreichendem Maße möglich ist, einen ausreichenden und trotzdem lokal begrenzten Energieeintrag in eine ausgedehnte Zielstruktur innerhalb der Haut oder des subkutanen Gewebes zu realisieren.

Ausgehend von dem aus dem Stand der Technik bekannten technischen Lösungen sowie den zuvor geschilderten Problemen liegt der Erfindung die Aufgabe zu Grunde, eine Vorrichtung zur Behandlung der Haut eines Menschen oder eines Tieres mittels eines Lasers derart weiterzubilden, dass der benötigte Energieeintrag zumindest nahezu ausschließlich in die zu schädigende Zielstruktur erfolgt. Hierbei soll insbesondere eine Schädigung der Epidermis der Haut, auf die Laserstrahlung auftritt, vermieden werden und insgesamt der Energieeintrag in die Haut bzw. das Gewebe auf das jeweils benötigte Mindestmaß reduziert werden. Das beschriebene System soll sich weiterhin auf vergleichsweise einfache Weise in ein kompaktes Gerät integrieren lassen, und einen wirtschaftlichen Einsatz im täglichen Betrieb ermöglichen. Weiterhin soll das anzugebende System derart ausgeführt werden, dass ein flexibler Einsatz, insbesondere die Durchführung einer Vielzahl von unterschiedlichen Behandlungen der Haut und des darunter liegenden Gewebes möglich ist.

Die zuvor beschriebene Aufgabe wird mit einer Vorrichtung gemäß Anspruch 1 gelöst. Ein geeignetes Verfahren zur Erzeugung eines Brennpunktes oder eines Brennflecks in einer Zielstruktur ist im Anspruch 12 sowie eine auf der Erfindung beruhende Verwendung eines entsprechend erzeugten Laserstrahls ist in Anspruch 13 angegeben. Vorteilhafte Ausführungsformen der Erfindung sind in den abhängigen Ansprüchen angegeben und werden in der folgenden Beschreibung unter teilweiser Bezugnahme auf die Figuren näher erläutert.

Die Erfindung betrifft eine Vorrichtung zur Bestrahlung einer Zielstruktur, die sich innerhalb von menschlicher oder tierischer Haut oder Gewebe, insbesondere subkutanem Gewebe, befindet. Die Vorrichtung verfügt über wenigstens eine Laserlichtquelle und zumindest ein Optikelement, mittels dem die Laserstrahlung durch eine Lichtaustrittsöffnung geleitet und in einem Brennpunkt außerhalb der Lichtaustrittsöffnung fokussiert wird. Weiterhin ist wenigstens ein Stellelement vorgesehen, mit dem der Abstand zwischen einer Hautoberfläche und dem im Bereich der Zielstruktur positionierten Brennpunkt einstellbar ist. Erfindungsgemäß ist die Vorrichtung derart weitergebildet worden, dass das Optikelement eine Anordnung eines konvexen Spiegels innerhalb eines Hohlspiegels aufweist, die derart angeordnet sind, dass von der Laserlichtquelle emittierte Laserstrahlung zumindest teilweise durch eine Öffnung im Hohlspiegel hindurchtritt, die durch die Öffnung hindurchgetretene Laserstrahlung auf den konvexen Spiegel trifft, vom konvexen Siegel zum Hohlspiegel gelenkt wird und vom Hohlspiegel kommend die Austrittsöffnung durchquert.

Die erfindungsgemäße Anordnung eines konvexes Spiegels innerhalb eines Hohlspiegels entspricht im Wesentlichen der Ausgestaltung eines Schwarzschild-Objektives, wobei allerdings der Strahlengang des Laserstrahls von der Laserlichtquelle bis zur Austrittsöffnung und weiter zum Brennpunkt des Hohlspiegels verläuft. Das erfindungsgemäß verwendete Optikelement verfügt somit über zwei einander gegenüber liegende, zentrisch angeordnete Spiegel bzw. Reflektorelemente. Der Hauptspiegel ist als konkaver Hohlspiegel konzipiert, dessen Spiegelfläche in Richtung der Lichtaustrittsöffnung ausgerichtet ist. Der deutlich kleinere, konvex geformte Spiegel, auch Auffangspiegel bzw. Auffangreflektor genannt, liegt als Wölbspiegel in einer Position nahe der Lichtaustrittsöffnung, wobei seine Spiegelfläche zum Hauptspiegel weist.

Das von der Laserlichtquelle ausgesandte Laserlicht fällt somit durch eine Öffnung im Hohlspiegel auf den kleinen Auffangspiegel, wird von diesem in radialer bzw. kugel- oder halbkugelförmiger Richtung auf den Hohlspiegel umgelenkt, der die Laserstrahlung schließlich durch die Austrittsöffnung in die Umgebung der Vorrichtung leitet.

In diesem Zusammenhang ist es denkbar, dass der Hohlspiegel entweder als Parabolspiegel oder als sphärischer Hohlspiegel ausgeführt ist. Hierbei zeichnet sich ein Parabolspiegel dadurch aus, dass alle Lichtstrahlen, die parallel zur optischen Achse auf den Spiegel auftreffen exakt im Brennpunkt gebündelt werden. Ein sphärischer Hohlspiegel, der die Form eines Kugelausschnitts aufweist, ist dagegen vergleichsweise einfach und damit kostengünstig herzustellen. Grundsätzlich ist es von Vorteil, den Hohlspiegel einstückig auszuführen. Generell ist aber auch eine mehrteilige, insbesondere zweiteilige Ausgestaltung denkbar, wobei in jedem Fall vorzugsweise mittig eine Öffnung vorzusehen ist, durch die die von der Laserlichtquelle kommende Strahlung hindurchtreten kann, um auf den konvexen Spiegel auftreffen zu können.
Der Mittelpunkt des konvexen Spiegels, insbesondere die der Laserlichtquelle am nächsten liegende Stelle, befindet sich bevorzugt auf der optischen Achse des von der Laserlichtquelle emittierten Laserstrahls.

Um den Brennpunkt innerhalb der Zielstruktur zu positionieren, muss die Laserlichtquelle mit dem Optikelement in einem entsprechenden Abstand zur Zielstruktur überführt werden. Oftmals befindet sich die Zielstruktur nicht direkt auf der Hautoberfläche, sondern liegt unterhalb der Hautoberfläche, etwa im subkutanen Gewebe. Entsprechend der Lage der Zielstruktur muss die Eindringtiefe der für die Behandlung vorgesehenen Laserstrahlung und hier insbesondere der Abstand des Brennpunktes von der Hautoberfläche eingestellt werden. Unter Eindringtiefe der Strahlung wird in diesem Zusammenhang regelmäßig die Tiefe des Brennpunkts innerhalb des Körpers des Patienten, also der Abstand des Brennpunktes von der Hautoberfläche verstanden.

Sofern sich die Zielstruktur nicht unmittelbar auf der Hautoberfläche befindet, hat dies in Bezug auf die auf die Hautoberfläche auftreffende Laserstrahlung den Effekt, dass die Auftrittsfläche auf der Hautoberfläche eine Kreisring- oder Ellipsenform aufweist, während die Strahlung im Brennpunkt, der sich dann unterhalb der Hautoberfläche in der Zielstruktur befindet, fokussiert wird. Der Energieeintrag auf die Hautoberfläche, die nicht behandelt werden soll und für die eine nachhaltige Schädigung verhindert werden soll, wird somit minimiert. Dagegen wird der maximale Energieeintrag innerhalb der Zielstruktur, nämlich im Brennpunkt erreicht. In diesem Zusammenhang wird darauf hingewiesen, dass die Strahlung gemäß der Erfindung regelmäßig nicht exakt in einem Brennpunkt sondern vielmehr in einem Brennbereich oder Brennvolumen fokussiert wird, wobei in diesem Bereich die Intensität der eingestrahlten Laserstrahlung maximal ist.

Wesentlich für die Positionierung des Brennpunktes bzw. des Brennbereiches in der Zielstruktur ist somit das erfindungsgemäß vorgesehene Stellelement, mit dem der Abstand zwischen einer Hautoberfläche und dem im Bereich der Zielstruktur positionierten Brennpunkt gezielt einstellbar ist. In der einfachsten Ausgestaltung wird diese Einstellung dadurch erreicht, dass die Laserlichtquelle mit dem Optikelement und der Austrittsöffnung, die bevorzugt in und/oder an einem Gerätegehäuse angeordnet sind, manuell oder durch einen Roboterarm in eine Position überführt wird, sodass der Brennpunkt bzw. der Brennbereich innerhalb der Zielstruktur zu liegen kommt Um den benötigten Abstand zur Hautoberfläche einstellen zu können ist ein Stellelement vorgesehen, mit dem der Abstand zwischen einer Hautoberfläche und dem im Bereich der Zielstruktur positionierten Brennpunkt gezielt einstellbar ist.

In einer speziellen Weiterbildung der Erfindung ist das Stellelement als Abstandhalter ausgebildet, der den jeweils benötigten Abstand zwischen der Hautoberfläche und der Lichtaustrittsöffnung einstellt. Hierfür ist wesentlich, dass durch eine Veränderung des Abstandes zwischen der Lichtaustrittsöffnung und der Hautoberfläche, zumindest bei unveränderten Optikeinstellungen, gleichzeitig der Abstand zwischen der Hautoberfläche und dem Brennpunkt verändert wird. Auf diese Weise kann durch Wahl des geeigneten Abstandhalters die Eindringtiefe des Brennpunktes der Strahlung innerhalb der Haut bzw. dem unterhalb der Haut gelegenen Gewebe verändert werden. Grundsätzlich ist es in diesem Zusammenhang denkbar, eine Mehrzahl von Abstandhaltern unterschiedlicher Größe, insbesondere unterschiedlicher Länge vorzusehen, die beispielsweise an einem Gehäuse der erfindungsgemäß ausgeführten Vorrichtung austauschbar befestigt werden.

In einer weiteren Ausführungsform der Erfindung ist vorgesehen, dass der Abstandhalter einen Aktuator aufweist, über den der Abstandhalter wenigstens teilweise derart bewegbar ist, dass wenigstens zwei unterschiedliche Abstände zwischen der Austrittsöffnung und der Haut einstellbar sind. Ein derartiger Aktuator kann beispielsweise in Form eines Hebels oder Stellrades mit daran gekoppelten bewegbaren Elementen ausgeführt sein, sodass eine Länge des Abstandhalters auf den gewünschten Abstand zwischen Lichtaustrittsöffnung und Hautoberfläche einstellbar ist. Alternativ oder in Ergänzung ist es denkbar, dass der Aktuator bevorzugt elektrisch angetriebene Elemente, beispielsweise einen Elektromotor, der bei Bedarf mit einem Getriebe kombiniert werden kann, aufweist, über die der Abstandhalter auf die entsprechende Länge ausgefahren werden kann, bzw. mit denen der benötigte Abstand zwischen der Lichtaustrittsöffnung und der Hautoberfläche eingestellt werden kann.

Eine weitere Ausführungsform der Erfindung sieht vor, dass das Stellelement wenigstens ein Bewegungsmittel aufweist, mit dem wenigstens ein Bauteil des Optikelementes derart bewegbar ist, dass der Abstand zwischen der Austrittsöffnung und dem Brennpunkt aufgrund der Bewegung verstellt wird. Diese Ausführungsform bietet den Vorteil, dass die Vorrichtung in gleichbleibendem Abstand auf die Hautoberfläche aufgesetzt werden kann und die Eindringtiefe des Brennpunktes innerhalb der Haut durch eine Verstellung wenigstens eines Bauteils des Optikelementes verstellbar ist. Bevorzugt ist das Bewegungsmittel mit wenigstens einem der beiden Spiegel des Optikelementes mechanisch verbunden und kann wenigstens einen dieser Spiegel derart bewegen, dass die Eindringtiefe der Laserstrahlung auf den benötigten Wert eingestellt werden kann. Selbstverständlich ist auch der Einsatz pneumatisch oder elektromagnetisch angetriebener Bewegungsmittel denkbar.

In einer besonderen Ausführungsform ist ferner zumindest ein Kühlelement vorgesehen, mit dem eine Hautoberfläche, insbesondere eine Oberfläche der Hornschicht, kühlbar ist. Der Einsatz eines derartigen Kühlelementes, mit dem die Hautoberfläche, auf die die Laserstrahlung auftrifft, gezielt gekühlt wird, bietet den Vorteil, dass einer Erwärmung der Haut durch die auftreffende Laserstrahlung entgegen gewirkt werden kann. In diesem Zusammenhang ist es auf vorteilhafte Weise denkbar, dass die Kühlung der Haut geregelt erfolgt, wobei für die Regelung der Kühlung die jeweilige Leistung des von der Laserlichtquelle ausgesandten Laserlichts berücksichtigt wird.

Weiterhin bevorzugt ist eine Steuereinheit vorgesehen, die zumindest zeitweise ein Steuersignal an die Laserlichtquelle und/oder das Stellelement überträgt. Mit einer derartigen Steuereinheit ist es möglich, einerseits die Intensität der von der Laserlichtquelle ausgesandten Laserstrahlung und andererseits die Eindringtiefe der Laserstrahlung in die Haut bzw. in das subkutane Gewebe gezielt zu verändern und so an die patientenspezifischen Bedürfnisse bzw. die geplante Behandlung anzupassen.

Ferner ist vorzugsweise ein Datenspeicher vorgesehen, der sich innerhalb der Steuereinheit befindet oder zumindest mit dieser verbunden ist, und in dem Eigenschaften des zu behandelnden bzw. des zu be- oder durchstrahlenden Gewebes abgelegt sind. Insbesondere sind hier optische Eigenschaften verschiedener Haut- und/oder Gewebebereiche oder entsprechender Haut- und/oder Gewebetypen abgelegt, sodass die Laserlichtquelle und/oder das Optikelement mit Hilfe eines von der Steuereinheit erzeugten Steuersignals derart eingestellt werden können, dass eine gezielte Behandlung der Zielstruktur erfolgt, ohne das umliegende, nicht zu behandelnde Gewebe nachhaltig geschädigt werden.

Bevorzugt werden Werte für spezielle optische Eigenschaften, wie beispielsweise der Brechungsindex und/oder das Absorptionsvermögen verschiedenartiger Haut- oder Gewebetypen in dem Datenspeicher abgelegt. Auf vorteilhafte Weise können die benötigten Werte vor Behandlungsbeginn vom Anwender ausgewählt und automatisch der Ansteuerung der Laserlichtquelle und/oder des Optikelements zu Grunde gelegt werden.

In einer speziellen Weiterbildung der Erfindung handelt es sich bei der Laserlichtquelle um eine Lichtquelle, die hinsichtlich der Intensität und/oder der emittierten Lichtwellenlänge geregelt werden kann. Bevorzugt weist die Laserlichtquelle einen He-Ne-Laser, einen Nd-YAG-Laser und/oder einen Er-YAG-Laser.

Gemäß einer besonderen Ausgestaltung der Erfindung ist vorgesehen, dass wenigstens zwei, vorzugsweise eine noch größere Mehrzahl von Laserquellen verwendet wird, um einerseits den zu behandelnden Bereich zu vergrößern und/oder andererseits unterschiedliche potentielle Wegstrecken vorzusehen, über die die Laserlichtstrahlung durch die Haut und den Körper des Patienten in den zu behandelnden Bereich gelangt. In diesem Zusammenhang ist es vorteilhaft, wenn den entsprechenden Laserlichtquellen ein erfindungsgemäß ausgeführtes Optikelement mit den beiden zuvor beschriebenen Spiegeln zugeordnet ist. Weiterhin ist es von Vorteil, wenn zumindest eine der Laserlichtquellen und/oder das jeweils zugeordnete Optikelement bewegbar ausgeführt sind. Die Bewegung erfolgt beispielsweise mit einem geeigneten Stellmotor, der durch die Steuereinheit auf der Art und des Fortschritts der Behandlung angesteuert wird. In einer ganz besonderen Weiterbildung ist vorgesehen, eine Mehrzahl von Laserlichtquellen mit daran befestigten Optikelementen vorzusehen, auf einem Kreis oder Kreisabschnitt angeordnet und an einem entsprechenden Gestell befestigt sind. Hierbei können wahlweise das Gestell und/oder die daran befestigten Baugruppe, zumindest bestehend aus Laserlichtquelle und Optikelement, bewegbar ausgeführt sein.

Vorzugsweise befinden sich die zuvor beschriebenen Bauteile einer erfindungsgemäß ausgeführten Vorrichtung in einem Gerätegehäuse, das zumindest einen Handgriff zur manuellen Führung und Betätigung der Vorrichtung aufweist. Vorzugsweise ist im Bereich des Griffes wenigstens ein Schaltelement vorgesehen, durch das die Laserlichtquelle eingeschaltet und/oder gewünschte Parameter, insbesondere als Führungs- oder Sollwerte eingegeben werden können. Gemäß einer speziellen Weiterbildung der Erfindung können durch dieses Schaltelement oder ein weiteres Schaltelement auch gezielt Einstellungen des Stellelements und/oder des Optikelements verändert werden, so dass ein Abstand zwischen der Lichtaustrittsöffnung und dem Brennpunkt auf den benötigten Wert einstellbar ist.

Weiterhin bevorzugt verfügt das Gerätegehäuse über wenigstens eine Anzeigeeinheit, insbesondere ein Display, zur Anzeige von im Datenspeicher abgelegten, auswählbaren Parametern, während der Verwendung der Vorrichtung Istwerten auftretenden und/oder voreingestellten oder eingegebenen Sollwerten.

Neben einer Vorrichtung betrifft die Erfindung auch ein Verfahren zur Erzeugung eines Brennflecks oder Brennpunkts zur gezielten Veränderung wenigstens einer Materialeigenschaft eines Bauteils in einer Zielstruktur. Bei Verwirklichung des erfindungsgemäßen ergibt sich die im Folgenden angegebene Reigenfolge von Verfahrensschritten:
- Erzeugen einer Laserstrahlung mit einer Laserlichtlichtquelle und Lenkung der Laserstrahlung mit Hilfe eines Optikelement zu einer Lichtaustrittsöffnung, sodass die Strahlung außerhalb der Lichtaustrittsöffnung in dem Brennpunkt oder Brennfleck fokussiert wird und
- Einstellen eines Abstands zwischen einer Bauteiloberfläche und dem im Bereich der Zielstruktur positionierten Brennfleck oder Brennpunkt mittels eines Stellelements.

Das erfindungsgemäße Verfahren zeichnet sich dadurch aus, dass die von der Laserlichtquelle emittierte Laserstrahlung zumindest teilweise von einer Rückseite kommend, die einer Spiegelfläche gegenüber liegt, durch eine Öffnung in einem Hohlspiegel hindurchtritt, die durch die Öffnung hindurchgetretene Laserstrahlung einen konvexen Spiegel gelenkt wird, vom konvexen Siegel auf die Spiegelfläche des Hohlspiegels geleitet wird und vom Hohlspiegel kommend durch die Austrittsöffnung zum Brennfleck gelenkt wird.

Mit Hilfe der zuvor beschriebenen Vorrichtung sowie des Verfahrens können auf bevorzugte Weise Eigenschaftsveränderungen eines Materials in einer Zielstruktur, insbesondere in einer Zielstruktur, die in der Haut und/oder in subkutanem Gewebe eines Menschen oder eines Tieres liegt, verändert werden. In diesem Zusammenhang lässt sich die erfindungsgemäße Vorrichtung und insbesondere die von dieser Vorrichtung im Bereich des Brennpunktes oder Brennflecks erzeugte Laserstrahlung zur Behandlung von Akne, Hautverunreinigungen, Hämangiomen, Zellulitis, Hyperhidrose, Hautkrebs, Hautfalten, Varicosis, Bandscheibenprolaps und/oder Fett verwenden.

Im Folgenden wird die Erfindung ohne Beschränkung des allgemeinen Erfindungsgedankens anhand eines Ausführungsbeispiels unter Bezugnahme auf die Figuren näher erläutert.

Dabei zeigen:
- Figur 1:: Handführbares Gerät mit einer erfindungsgemäß ausgeführten Vorrichtung zur Erzeugung eines in eine Zielstruktur gerichteten Laserstrahls;
- Figur 2:: schematische Darstellung einer Laserlichtquelle mit einem Optikelement, das eine erfindungsgemäße Strahlführung ermöglicht sowie
- Figur 3:: Anordnung zur Durchführung einer dermatologischen Behandlung mit einer Mehrzahl von eine Laserlichtquelle und ein Optikelement aufweisenden Baugruppen.

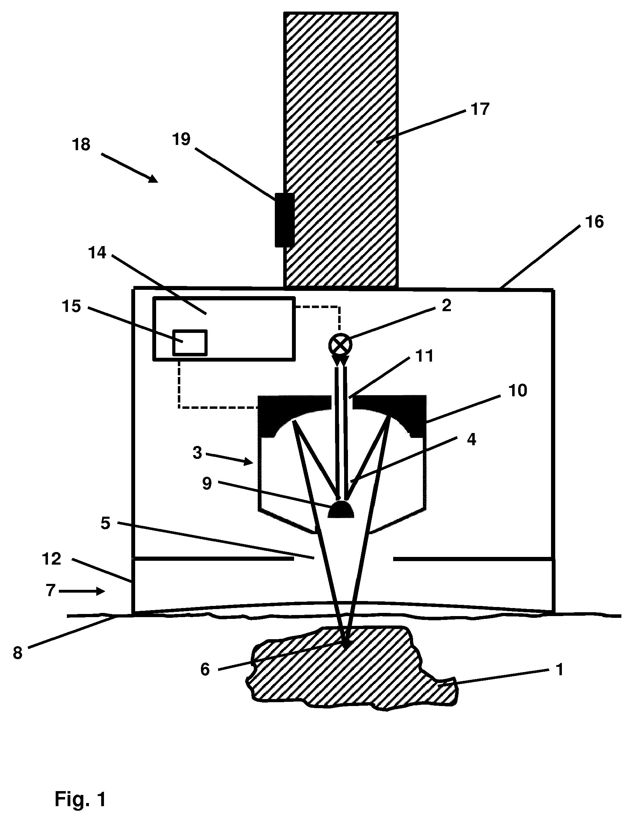

Figur 1 zeigt eine erfindungsgemäße Vorrichtung, die als handführbares Gerät 18 zur Durchführung von dermatologischen Behandlungen mittels Laserlicht 4 ausgebildet ist. Das Bestrahlungsgerät 18 kann mit der Hand bedient und geführt werden und wird zur Behandlung über den zu bestrahlenden Bereich mit der Zielstruktur 1 gehalten. Das dargestellte Bestrahlungsgerät 18 verfügt im Wesentlichen über einen Gehäusekörper 16, in dem eine Laserlichtquelle 2, ein Optikelement 3 und eine Lichtaustrittsöffnung 5 angeordnet sind, sowie über ein Stellelement 7 ein Handgriff 17 zur Führung des Geräts, die am Gehäuse 16 befestigt sind. Im Bereich des Handgriffs 17 ist als Schaltelement 19 ein Handschalter vorgesehen, über das die Laserlichtquelle 2 ein und ausgeschaltet werden kann.

Mit dem dargestellten Handbestrahlungsgerät 18 ist es möglich, auf einfache Weise eine Behandlung von kranker Haut oder subkutanem Gewebe durchzuführen. Innerhalb des Gerätegehäuses 16 ist eine Laserlichtquelle 2 zur Erzeugung von Laserstrahlung 4 vorgesehen. Der Laserstrahl 4 wir durch eine Öffnung 11 in einem Hohlspiegel 10 von dessen Rückseite kommend auf einen konvexen Spiegel 9 geleitet, der im Inneren des Hohlspiegels 10 auf der optischen Achse der Laserstrahlung 4 angeordnet ist. Von diesem konvexen Spiegel 9 wird die Laserstrahlung 4 auf die Spiegelfläche des Hohlspiegels 10 und von dort durch die in der Wand des Gehäuses 16 vorgesehenen Lichtaustrittsöffnung 5 gelenkt, so dass die Strahlung 4 im Bereich des Brennpunktes oder Brennflecks 6 fokussiert wird.

Als Stellelement 7 ist ein austauschbar am Gehäuse befestigter Abstandhalter 12 vorgesehen, der auf die Hautoberfläche 8 des Patienten oberhalb der Zielstruktur 1 aufgesetzt wird und einen festen Abstand zwischen der Lichtaustrittsöffnung 5 und der Hautoberfläche 8 festlegt, sodass auch der Abstand zwischen Hautoberfläche 8 und Brennpunkt oder Brennfleck 6 einen bestimmten, dem Nutzer bekannten Abstand aufweist. Hierbei sind auf vorteilhafte Weise unterschiedliche Abstandhalter 18 vorhanden, die in Abhängigkeit des Bedarfs, insbesondere in Abhängigkeit der benötigten Eindringtiefe in die Haut oder in das darunter befindliche Gewebe, wechselweise am Gehäuse 16 des Bestrahlungsgeräts 18 befestigt werden können und jeweils einen vorgegebenen Abstand zwischen der Lichtaustrittsöffnung 5 und der Hautoberfläche 8 einstellen. Ebenso ist es denkbar, ein Stellelement 7 vorzusehen, das am Gehäuse 16 des Bestrahlungsgeräts 18 befestigt ist und das über bewegbare Bauteile verfügt, so dass der Abstand zwischen der Lichtaustrittsöffnung 5 und der Hautoberfläche 8 bedarfsgerecht und gezielt eingestellt werden kann. Eine Verstellung kann manuell, bspw. mit Hilfe eines Schraubmechanismus, oder mit Hilfe eines Antriebselements, wie etwa einem Elektromotor, erfolgen.

Der maximale Außendurchmesser des Handbestrahlungsgeräts 18 im Bereich des Gehäusekörpers 16 beträgt etwa 6 cm. Bei einer besonderen konstruktiven Gestaltung liegt dieser Durchmesser bei etwa 3,5 bis 5 cm.

In der Steuerung 14, in der Steuersignale zum Betrieb der Laserlichtquelle 2 erzeugt werden, sind in einem Datenspeicher 15 optische Eigenschaften des zu be- und durchstrahlenden Gewebes und der Haut hinterlegt. Vor Behandlungsbeginn wird vom Anwender ein Behandlungsbereich innerhalb der Haut oder des subkutanen Gewebes lokalisiert und eine geeignete dreidimensionaler Zielstruktur 1 festgelegt, die bestrahlt werden soll. Im Weiteren werden für die Bestrahlung die festgelegten Strahlungsparameter der Laserstrahlung 4 sowie die in der Steuerung 14 hinterlegten optischen Eigenschaften der durchstrahlten Bereiche, unter anderem der Hautoberfläche 8, einerseits und der Zielstruktur 8 andererseits berücksichtigt. Auf diese Weise wird sichergestellt, dass während der Behandlung in der Haut bzw. im subkutanen Gewebe zumindest ein räumlicher Brennfleck 6 erzeugt wird, in dem die gewünschten Eigenschaftsveränderungen bewirkt werden.

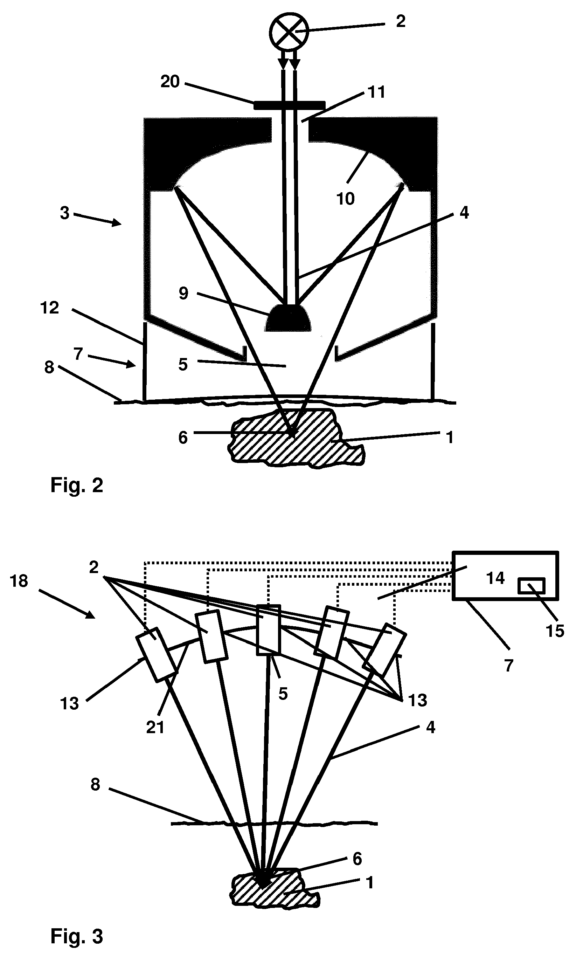

In Figur 2 wird ist eine schematische Darstellung einer Laserlichtquelle 2 mit einem Optikelement 3, das eine erfindungsgemäße Strahlführung ermöglicht, gezeigt. Mit Hilfe der dargestellten Vorrichtung wird innerhalb der Zielstruktur 1 ein Brennpunkt oder ein Brennfleck 6 mit räumlicher Ausdehnung erzeugt. Wesentlich ist in diesem Fall, dass die von der wenigstens einen Laserlichtquelle 2 ausgesandte Strahlung 4 mit Hilfe eines Hohlspiegels 10 und einem mittig in dem Hohlspiegel angeordneten Konvexspiegel 9, die wie bei einem Schwarzschild-Objektiv angeordnet sind, auf den zu behandelnden Bereich, insbesondere in die Zielstruktur 1 gelenkt wird.

Die in diesem Fall für das Optikelemente verwendete Anordnung aus Hohlspiegel 10 und Konvexspiegel 9 zeichnet sich vor allem dadurch aus, dass ein Lichtstrahl 4, der eine inhomogene Intensitätsverteilung mit höherer Intensität in der Strahlmitte als am Strahlrand aufweist, wie es beispielsweise bei Lichtstrahlen, die von Excimerlasern emittiert werden, der Fall ist, verkleinert abgebildet wird. Excimerlaser emittieren kohärente UV-Lichtstrahlen hoher Intensität und werden daher vielfach in der Medizin eingesetzt.

Von großer Bedeutung für die erfindungsgemäße Anordnung der Spiegel 9, 10 des Optikelements 3 sind die beiden einander gegenüberliegend, zentrisch angeordneten Spiegel 9, 10. Der Hauptspiegel ist als Hohlspiegel 10 konzipiert, dessen Spiegelfläche zur Lichtaustrittsöffnung 5 bzw. zur Hautoberfläche 8 gerichtet ist. Der deutlich kleinere Auffangspiegel 9 liegt als Wölb- bzw. Konvexspiegel in Richtung zur Lichtaustrittsöffnung 5, wobei seine Spiegelfläche zum Hauptspiegel 10 zeigt.

Die von der Laserlichtquelle 2 emittierten Lichtstrahlen 4 gelangen zunächst in eine Diffusoroptik 20, durch die eine Intensitätsverteilung des Lichtstrahls 4 vor Eintritt in die Anordnung aus Hohl- 10 und Konvexspiegel 9 derart verändert wird, dass der Lichtstrahl 4 in der Mitte eine geringere Intensität als in den Randbereichen aufweist. Die Diffusoroptik 20 kann hierfür etwa über ein Biprisma und einen Homogenisierer verfügen. Anschließend werden die Lichtstrahlen 4 mit Hilfe des konvexen Auffangspiegels 9 und des Hohlspiegels 10 auf die festgelegte Zielstruktur 1 fokussiert.

Wesentlich für die gezeigte technische Lösung ist, dass die Laserstrahlung 4 im Bereich der Zielstruktur 1 fokussiert wird, sodass die Strahlungsintensität im Bereich eines in der Zielstruktur 1 liegenden Brennflecks 6 ein Maximum annimmt, und dass dagegen diejenigen Haut- und Gewebebereiche, die nicht nachhaltig verändert werden sollen, nur einer vergleichsweise geringen Strahlungsleistung bzw. Strahlendosis ausgesetzt werden. Dies ist auf die besondere Strahlführung zurückzuführen, bei der die Strahlung 4 insbesondere im Bereich der Hautoberfläche 8 auf eine vergleichsweise große Kreisringfläche verteilt wird. Eine Fokussierung der Laserstrahlung 4 kann somit zielgenau in der Zielstruktur 1 erfolgen.

Um in speziellen Fällen eine zusätzliche Maßnahme zum Schutz der Haut, auf die zumindest zeitweise ein Teil der Laserstrahlung auftrifft, vorzusehen, ist in Abhängigkeit der gewählten Behandlung ein Kühlelement vorgesehen, das die Hautoberfläche 8 kühlt und so zusätzlich vor einer ungewünschten Schädigung durch die Laserbehandlung schützt.

Figur 3 zeigt eine spezielle Anordnung von Baugruppen, die jeweils eine Laserlichtquelle 2 und ein Optikelement 3 mit zwei Spiegeln, die, wie von einem Schwarzschild-Objektiv bekannt, angeordnet sind, aufweisen. Die Laserlichtquellen 2 mit den diesen nachgeschalteten Optikelementen 3 sind an einem Geräteträger 21 befestigt, wobei jeweils Bewegungsmittel 13 mit Stellmotoren vorgesehen sind, die auf der Grundlage eines von einer zentralen Steuereinheit 14 erzeugten Steuersignals die Laserlichtquellen 2 mit den Optikelementen 3 bedarfsgerecht bewegen. Mit Hilfe einer derartigen Anordnung mehrerer Lichtquellen 2, die gemäß dem in Figur 3 gezeigten Ausführungsbeispiel angeordnet sind, ist es einerseits möglich in einer Behandlungsphase eine besonders großflächige Bestrahlung durchzuführen, anderseits wird es so ermöglicht, Laserlichtstrahlen 4 auf unterschiedlichen Strahlungswegen in den Körper einzustrahlen. Durch diese Maßnahme kann wahlweise die Intensität einer Bestrahlung während einer Behandlung über einen großen Bereich variiert werden, es können zeitgleich unterschiedliche Bereiche einer Zielstruktur bestrahlt werden und/oder die Strahlungsbelastung der oberen Hautschichten kann auf geeignete Weise begrenzt werden, ohne dass es zu Verzögerungen bei der Behandlung kommt.

Figur 3 zeigt eine schematische Darstellung einer Anordnung bestehend aus einer Mehrzahl von bewegbaren Baugruppen jeweils mit Laserlichtquelle 2 und Optikeinheit 3. Die Optikeinheiten 3 verfügen, wie im Zusammenhang mit den Figuren 1 und 2 bereits erläutert, über einen Hohlspiegel 10 und einen konvexen Auffangspiegel 9, deren Spiegeloberflächen einander zugewandt sind und die eine Strahlführung, wie sie etwa aus Schwarzschild-Objektiven bekannt ist, ermöglichen. Die hierfür vorgesehene Steuereinheit 14 erzeugt auf der Grundlage der vom Anwender eingegebenen Daten und/oder von Parametern, die in einem Datenspeicher 15 abgelegt sind und Informationen über Haut, Gewebe und Strahleneigenschaften enthalten können, Steuersignale um die Laserlichteinstellen und einen Behandlungserfolg bei minimalen Schädigungen des gesunden Gewebes und der gesunden Haut sicher zu stellen. In diesem Zusammenhang wird mittels der zentralen Steuereinheit 14 auch der benötigte Abstand zwischen den Lichtaustrittsöffnungen 5 und der Hautoberfläche 8 eingestellt. Um eine geeignete Einstellung zu bewirken, übernimmt die Steuereinheit 14 zusammen mit entsprechend geeigneten Bewegungsmittel 13 zur gesteuerten Bewegung des Geräteträgers 21, der Laserlichtquellen 2 und/oder der Optikelement 3, insbesondere der Spiegel 9, 10 die Funktion des Stellmittels 7. Eine derartige automatisierte Einstellung unterschiedlicher Komponenten und damit des Abstandes zwischen den Lichtaustrittsöffnungen 5 und der Hautoberfläche 8 und damit auch zwischen der Hautoberfläche 8 und der Zielstruktur 1, um so einen Brennpunkt oder Brennfleck innerhalb der Zielstruktur 1 zu erzeugen, erfolgt bevorzugt mit Hilfe eines stationären Bestrahlungsgerätes.

Ebenso ist es möglich, eine derartige Anordnung zu miniaturisieren, so dass die Anordnung in einem handführbaren Gerät 18 Platz findet. Wesentlich ist jeweils, dass mit einer entsprechenden Anordnung, unabhängig von ihrer Baugröße entweder zeitgleich, zeitlich überlappend oder in zeitlichem Abstand zu einander wenigstens zwei Laserstrahlen 4 erzeugt werden, die wenigstens abschnittsweise auf unterschiedlichen Wegen oder zu unterschiedlichen Zeitpunkten den Körper des Patienten in Richtung der Zielstruktur 1 durchlaufen. Sofern eine entsprechende Anordnung in einem handführbaren Gerät verbaut wird, ist es von Vorteil, wenn dem Behandler, insbesondere über ein externes oder in das Handgerät integrierte Display, eine Information darüber ausgegeben wird, ob sich Gerät in der richtigen Position relativ zur Zielstruktur 1, insbesondere im richtigen Abstand zur Hautoberfläche 8 befindet. Die Information wird derart ausgegeben, dass es dem Benutzer auf einfache Weise möglich ist zu erkennen, ob und in welche Richtung das Gerät bewegt werden muss.

### Bezugszeichenliste

- 1: Zielstruktur
- 2: Laserlichtquelle
- 3: Optikelement
- 4: Laserlichtstrahlung
- 5: Lichtaustrittsöffnung
- 6: Brennpunkt / Brennfleck
- 7: Stellelement
- 8: Hautoberfläche
- 9: konvexer Spiegel
- 10: Hohlspiegel
- 11: Öffnung im Hohlspiegel
- 12: Abstandhalter
- 13: Bewegungsmittel
- 14: Steuereinheit
- 15: Datenspeicher
- 16: Gehäuse
- 17: Handgriff
- 18: Bestrahlungsgerät
- 19: Schaltelement
- 20: Diffusorelement
- 21: Geräteträger

## Patentansprüche

1. Vorrichtung zur Bestrahlung einer Zielstruktur (1), die sich innerhalb von menschlicher oder tierischer Haut oder Gewebe befindet, mit wenigstens einer Laserlichtquelle (2) und zumindest einem Optikelement (3), mittels dem die Laserstrahlung (4) durch eine Lichtaustrittsöffnung (5) geleitet und in einem Brennpunkt (6) außerhalb der Lichtaustrittsöffnung (5) fokussiert wird, wobei wenigstens ein Stellelement (7) vorgesehen ist, mit dem der Abstand zwischen einer Hautoberfläche (8) und dem im Bereich der Zielstruktur (1) positionierten Brennpunkt (6) einstellbar ist,
**dadurch gekennzeichnet, dass** das Optikelement (3) eine Anordnung eines konvexen Spiegels (9) innerhalb eines Hohlspiegels (10) aufweist, die derart angeordnet sind, dass von der Laserlichtquelle (2) emittierte Laserstrahlung (4) zumindest teilweise durch eine Öffnung (11) im Hohlspiegel (10) hindurchtritt, die durch die Öffnung (10) hindurchgetretene Laserstrahlung (4) auf den konvexen Spiegel (9) trifft, vom konvexen Siegel (9) zum Hohlspiegel (10) gelenkt wird und vom Hohlspiegel (10) kommend die Lichtaustrittsöffnung (5) durchquert.

2. Vorrichtung nach Anspruch 1,
**dadurch gekennzeichnet, dass** das Stellelement (7) über einen Abstandhalter (12) verfügt, der derart auf die Hautoberfläche (8) aufsetzbar ist, dass ein Abstand zwischen der Lichtaustrittsöffnung (5) und der Haut einstellbar ist.

3. Vorrichtung nach Anspruch 2,
**dadurch gekennzeichnet, dass** der Abstandhalter (12) einen Aktuator aufweist, über den der Abstandhalter (12) wenigstens teilweise derart bewegbar ist, dass wenigstens zwei unterschiedliche Abstände zwischen der Lichtaustrittsöffnung (5) und der Hautoberfläche (8) einstellbar sind.

4. Vorrichtung nach einem der Ansprüche 1 bis 3,
**dadurch gekennzeichnet, dass** das Stellelement (7) wenigstens ein Bewegungsmittel (13) aufweist, mit dem wenigstens ein Bauteil des Optikelements (3) derart bewegbar ist, dass der Abstand zwischen der Lichtaustrittsöffnung (5) und dem Brennpunkt (6) aufgrund der Bewegung verstellt wird.

5. Vorrichtung nach einem der Ansprüche 1 bis 4,
**dadurch gekennzeichnet, dass** zumindest ein Kühlelement vorgesehen ist, mit dem eine Hautoberfläche (8), insbesondere eine Oberfläche der Hornschicht, kühlbar ist.

6. Vorrichtung nach einem der Ansprüche 1 bis 5,
**dadurch gekennzeichnet, dass** eine Steuereinheit (14) vorgesehen ist, die zumindest zeitweise ein Steuersignal an die Laserlichtquelle (2), das Optikelement (3) und/oder das Stellelement (7) überträgt.

7. Vorrichtung nach Anspruch 6,
**dadurch gekennzeichnet, dass** in der Steuereinheit (14) oder einem an die Steuereinheit (14) gekoppelten Datenspeicher (15) Eigenschaften wenigstens einer Hautschicht und/oder eines Gewebetyps hinterlegt sind.

8. Vorrichtung nach Anspruch 6 oder 7,
**dadurch gekennzeichnet, dass** in der Steuereinheit (14) oder einem an die Steuereinheit (14) gekoppelten Datenspeicher (15) wenigstens eine optische Eigenschaft, insbesondere ein Wert für einen Brechungsindex und/oder für ein Absorptionsvermögen wenigstens eines Haut- und/oder Gewebebereichs hinterlegt ist.

9. Vorrichtung nach einem der Ansprüche 1 bis 8,
**dadurch gekennzeichnet, dass** die Laserlichtquelle (2) einen He-Ne-Laser, einen Nd-YAG-Laser und/oder einen Er-YAG-Laser aufweist.

10. Vorrichtung nach einem der Ansprüche 1 bis 9
**dadurch gekennzeichnet, dass** die Laserlichtquelle (2), das Optikelement (3) und das Stellelement (7) von einem einteiligen oder mehrteiligen Gehäuse (16) umgeben sind, in dem sich die Lichtaustrittsöffnung (5) befindet und an dem ein Handgriff (17) zur manuellen Führung befestigt ist.

11. Vorrichtung nach einem der Ansprüche 1 bis 10,
**dadurch gekennzeichnet, dass** zwischen der Laserlichtquelle (2) und dem konvexen Spiegel (9) eine Diffusoroptik (20) angeordnet ist, durch die eine Intensitätsverteilung der Laserlichtstrahlung (4) derart verändert wird, dass die Laserlichtstrahlung (4) in einer Strahlmitte eine geringere Intensität als im Randbereich aufweist.

12. Verfahren zur Erzeugung eines Brennflecks oder Brennpunkts (6) zur gezielten Veränderung wenigstens einer Materialeigenschaft eines Bauteils in einer Zielstruktur (1), bei dem mit wenigstens einer Laserlichtquelle (2) Laserstrahlung (4) erzeugt und von einem Optikelement (3), zumindest teilweise durch eine Lichtaustrittsöffnung (5) geleitet und in einem Brennpunkt (6) außerhalb der Lichtaustrittsöffnung (5)fokussiert, wobei mit Hilfe wenigstens eines Stellelements (7) ein Abstand zwischen einer Bauteiloberfläche und dem im Bereich der Zielstruktur (1) positionierten Brennfleck oder Brennpunkt (6) einstellbar ist,
**dadurch gekennzeichnet, dass** das die von der Laserlichtquelle (2) emittierte Laserstrahlung (4) zumindest teilweise von einer Rückseite kommend, die einer Spiegelfläche gegenüber liegt, durch eine Öffnung (11) in einem Hohlspiegel (10) hindurchtritt, die durch die Öffnung (11) hindurchgetretene Laserstrahlung (4) auf einen konvexen Spiegel (9) gelenkt wird, vom konvexen Siegel (9) auf die Spiegelfläche des Hohlspiegels (10) geleitet wird und vom Hohlspiegel (10) kommend durch die Lichtaustrittsöffnung (5) zum Brennfleck oder Brennpunkt (6) gelenkt wird.

13. Anordnung zur Bestrahlung einer Zielstruktur (1), die sich innerhalb von menschlicher oder tierischer Haut oder Gewebe befindet, mit wenigstens zwei Vorrichtungen gemäß einem der Ansprüche 1 bis 11.

14. Verwendung eines mit einer Vorrichtung gemäß einem der Ansprüche 1 bis 11, erzeugten Laserstrahls (4) zur Behandlung von Akne, Hautverunreinigungen, Hämangiomen, Zellulitis, Hyperhidrose, Hautkrebs, Hautfalten, Varicosis, Bandscheibenprolaps und/oder Fett.
